(19) Europäisches Patentamt / European Patent Office / Office européen des brevets

(11) EP 2 942 628 A1

(12) # EUROPEAN PATENT APPLICATION

(43) Date of publication:
**11.11.2015 Bulletin 2015/46**

(21) Application number: **15167043.7**

(22) Date of filing: **08.05.2015**

(51) Int Cl.:
*G01N 33/569* (2006.01) *C07K 14/005* (2006.01)
*C12N 7/00* (2006.01) *C12N 15/86* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **08.05.2014 IT TO20140366**

(71) Applicant: **In3diagnostic S.r.l.**
**10138 Torino (IT)**

(72) Inventors:
- **ROSATI, Sergio**
  **10020 Cambiano (IT)**
- **PROFITI, Margherita**
  **10072 Mappano (IT)**
- **BERTOLOTTI, Luigi**
  **Torino (IT)**

(74) Representative: **Bosia, Alessandra et al**
**STUDIO TORTA S.p.A.**
**Via Viotti, 9**
**10121 Torino (IT)**

(54) **Kit and in vitro method for identifying Bovine Herpes Virus 1 infections**

(57) The present invention relates to a kit comprising a support on which a recombinant bovine herpes virus 1 (BoHV1) glycoprotein E (gE) is immobilised. The recombinant BoHV1 gE comprises at least two different epitopes which respectively bind at least two different anti-glycoprotein E immunoglobulins. The present invention also relates to an in vitro method for immunologically identifying a bovine herpes virus 1 (BoHV1) infection comprising the steps of contacting an aliquot of a biological sample from a bovine or a plurality of bovines with the support of the kit in conditions allowing the formation of an immunological complex formed by the gE and immunoglobulins possibly present in the biological sample.

| | 1 | 2 | 3 | 4 | 5 | 6 | 7 | 8 | 9 | 10 | 11 | 12 |
|---|---|---|---|---|---|---|---|---|---|---|---|---|
| A | CNeg | CNeg | C7 | C7 | C15 | C15 | C23 | C23 | C31 | C31 | C39 | C39 |
| B | CPos | CPos | C8 | C8 | C16 | C16 | C24 | C24 | C32 | C32 | C40 | C40 |
| C | C1 | C1 | C9 | C9 | C17 | C17 | C25 | C25 | C33 | C33 | C41 | C41 |
| D | C2 | C2 | C10 | C10 | C18 | C18 | C26 | C26 | C34 | C34 | C42 | C42 |
| E | C3 | C3 | C11 | C11 | C19 | C19 | C27 | C27 | C35 | C35 | C43 | C43 |
| F | C4 | C4 | C12 | C12 | C20 | C20 | C28 | C28 | C36 | C36 | C44 | C44 |
| G | C5 | C5 | C13 | C13 | C21 | C21 | C29 | C29 | C37 | C37 | C45 | C45 |
| H | C6 | C6 | C14 | C14 | C22 | C22 | C30 | C30 | C38 | C38 | C46 | C46 |

FIG. 1

EP 2 942 628 A1

## Description

The present invention relates to a kit and to an in vitro method for identifying bovine herpes virus 1 infections in bovines.

### State of the art

Infectious bovine rhinotracheitis (IBR) is a viral and contagious disease caused by bovine herpes virus 1 (BoHV1), which belongs to the herpesviridae family and varicellovirus genus. The infection causes several clinical forms which have a heavy economical impact. Among these clinical forms: a respiratory form (rhinotracheitis), a genital form (pustular balanoposthitis and vulvovaginitis) and a reproductive form (embryonic death and resorption, abortion). The infection is spread worldwide. However, in many European countries it has been eradicated (Scandinavian countries, Denmark, Switzerland, Austria, province of Bolzano in Italy). In many other countries there are control programmes which are compulsory, voluntary or limited to some regions. The key to the success in eradicating the BoHV1 infection has been the introduction more than twenty years ago of a negative immunological marker vaccine, in which the gene codifying glycoprotein E is deleted. This vaccine provides immunity and at the same time allows to distinguish infected animals from vaccinated animals. This difference is detected by means of a serological test identifying antibodies directed to glycoprotein E (gE). Animals vaccinated with the marker vaccine are negative to the test because they do not have antibodies directed to gE, whereas the infected animals are positive to the test because all wild-type viral strains contain gE and induce the production of specific antibodies.

A serological diagnostic competitive test (blocking ELISA) has been developed together with the marker vaccine by using a monoclonal antibody directed to an immunodominant epitope of gE. In particular, this epitope is generated when the gE forms a complex with a second viral glycoprotein, glycoprotein I. The test therefore is based on the competition between the antibodies present in the sample serum and the monoclonal antibody directed to the same epitope. The above said vaccine and the above said serological diagnostic test, hereinafter referred to as "competitive ELISA" are disclosed in EP0587805.

The diagnostic test of EP0587805 has some disadvantages.

First, as it is a competitive test, it is based on the detection of antibodies directed to a single epitope. For this reason, the test sample must be rather concentrated (1:2 dilution) to block the epitope.

Further, the use of the test is limited to blood serum and its use on milk is not recommended by many laboratories because milk contains a reduced concentration of antibodies (about 1:15-1:20 of the antibodies present in blood serum). Some methods have been suggested to concentrate antibodies in milk, but the costs are too high for a routine use on individual samples.

Further, the competitive test produces a certain amount of false positives. These cases often occur in animals with high antibody titres directed to other viral glycoproteins (recently revaccinated animals), which block the binding between the monoclonal antibody and the epitope due to steric hindrance.

These disadvantages are accompanied by the fact that maintaining the IBR-free status certification for a farm requires a continuous control of the requirements through individual serological tests on all animals in the diagnostic age, according to the provisions of the current European Directive. This results in very high costs for many years after the health certification has been obtained.

Furthermore, there is currently no confirmation test as an alternative to the competitive ELISA which is also independent therefrom. The reason is that, when EP0587805 expired, many companies concentrated on the production of kits identical to the competitive kit.

The object of the present invention is to therefore provide a kit and a method allowing to identify a bovine herpes virus 1 (BoHV1) infection in a simple, fast, cost-effective manner. The kit and method need to be sensitive enough to allow use not only on serum samples but also with individual and pooled milk samples.

According to the present invention, this object is achieved by the kit according to claim 1.

According to the present invention, a method according to claim 9 is also provided.

### Definitions

Unless otherwise explicitly specified, the following terms have the following meaning.

By "glycoprotein E" or "gE" there is intended the bovine herpes virus 1 glycoprotein E unless otherwise specified.

By "secreted gE" there is intended the portion of the glycoprotein E in which the transmembrane domain has been deleted and which is released in the culture medium by mammal cells after transient transfection.

By "ELISA" there is intended Enzyme-Linked ImmunoSorbent Assay.

By "indirect ELISA" there is intended an ELISA kit or an ELISA method that involves immobilising the antigen on a solid phase, in which the reaction between antigen and antibody is detected with the addition of a secondary anti-species IgG reagent conjugated with an enzyme.

By "pooled milk" there is intended the milk obtained from the milking of a plurality of lactating animals.

By "concentrated milk IgG" there is intended a pool, or a mixture of isotype G immunoglobulins which are obtained by subjecting the pooled milk to a purification/concentration process.

Brief description of the figures

For a better understanding of the present invention, a preferred embodiment thereof will be disclosed hereinafter by mere way of non-limitative example with reference to the accompanying drawings, in which:

- Figure 1 is a diagrammatic representation of an ELISA plate according to a preferred embodiment of the kit according to the present invention;
- Figure 2 is a bar chart showing the results obtained with the kit and the method according to the present invention for pooled milk samples in which the milk positive for BoHV1 was increasingly diluted in milk negative for BoHV1;
- Figure 3 is a graph showing the reactivity in ELISA tests on concentrated pooled milk IgGs obtained in positive farms with increasing prevalence rates, assessed at the last annual control and referred to the lactating animals;
- Figure 4 is a bar chart in which each column represents the positive or negative result obtained by the kit and method according to the present invention on 64 samples of individual milk, pooled milk obtained by mixing the 64 samples, and pooled milk obtained by mixing the 64 samples and then concentrating the IgGs as disclosed in Example 2;
- Figure 5 is a graph showing the results of reactivity experiments on sera which were false positives to a competitive test according to the prior art;
- Figure 6 is a graph showing the results of seroconversion experiments in experimentally infected animals.

Detailed description of the invention

The kit according to the present invention comprises a support on which a recombinant bovine herpes virus 1 (BoHV1) glycoprotein E (gE) is immobilised. The recombinant glycoprotein E comprises at least two different epitopes binding respectively at least two different anti-gE immunoglobulins. The recombinant glycoprotein E preferably comprises at least three different epitopes binding respectively at least three different anti-gE immunoglobulins.

The support is preferably an ELISA microtitration plate, more preferably a 96 well ELISA microtitration plate, such as for example NUNC Maxisorp® plates.

The BoHV1 recombinant gE preferably comprises SEQ ID NO:1, more preferably is SEQ ID NO:1.

The BoHV1 recombinant gE is preferably produced in mammal cells. This allows to obtain a glycosylation pattern identical to that of the native antigen and ensures a higher antigenicity than with the antigen produced in cells from other organisms.

The amount of recombinant gE immobilised per well is preferably in the range from 0.025 $\mu$g to 0.2 $\mu$g, more preferably in the range from 0.05 $\mu$g to 0.1 $\mu$g.

Preferably, at least one other recombinant glycoprotein E is immobilised on the support, the at least one other recombinant glycoprotein E belonging to a herpes virus other than bovine herpes virus 1 (BoHV1). Non-limitative examples of the other recombinant glycoproteins E are: caprine herpes virus (CpHV), buffalo herpes virus (BuHV), equine herpes virus (EHV), ovine herpes virus (OvHV) etc. Furthermore, the glycoprotein E may be of different types of herpes virus: type 1, type 2 etc.

The kit may also comprise additional reagents for identifying the immunoglobulins and/or positive and/or negative control reagents, such as for example control sera.

A specific non-limitative example of the kit according to the present invention is disclosed hereinafter together with the preparation procedure thereof.

Production of the recombinant gE in secreted form

The gene codifying for the BoHV1 gE is synthetically generated by using the amino acid sequence SEQ ID NO:1 and using codons optimised for mammal cells. The amino acid sequence SEQ ID NO:1 lacks the leader peptide and the transmembrane dominium to allow the cloning in a eukaryotic expression vector under the control of the viral CMV promoter in-frame with the Murine Ig $\kappa$-chain leader sequence secretion signal. The cloned nucleotide sequence is SEQ ID NO:2. The vector must also comprise the SV40 promoter to allow the episomial replication of the plasmid in cells expressing the SV40 T large antigen. Purified preparations of the plasmid are obtained by using endotoxin free methods and are used to transiently transfect HEK293T cell lines.

A cationic lipid based method is used for transfection, but other methods may be used with similar results: cationic polymers (ex: PEI) or lyposomes. Six hours after transfection, the culture medium is replaced with protein free medium (ExCell293 Sigma Aldrich) and the cells are incubated up to 72 hours from transfection. The culture medium is harvested and used directly as antigen.

The kit must include the use of a negative control obtained by using the same conditioned medium after culture for 72 hours of the same cell line used for antigen production.

Preparation of the kit

The resulting antigen is used to produce an indirect ELISA test by immobilising the positive antigen (secreted gE expressed in HEK293T cells) in odd rows of a NUNC Maxisorp plate. The negative antigen (HEK293T culture medium) is immobilised at the same dilution in even rows of the same plate. Depending on the transfection efficiency, the antigen may be diluted starting from 1:10 by two-fold in 0.1 M carbonate/bicarbonate buffer pH 9.6. In the reported validation the antigen is diluted 1:10.

The ELISA plates are incubated at 4°C for 16 hours, blocked for 2 hours at 37°C by using a solution containing 2.5 % casein pH 7 and stabilised for 2 hours at room temperature with a 2 % saccharose solution in PBS. The plates are finally vacuum dried overnight and vacuum sealed in bags containing a 2 gram silica gel packet.

Content of the kit

2 ELISA plates prepared as disclosed above.
2x dilution buffer 1d: 1 25 ml bottle
20x washing buffer 2: 1 60 ml bottle
100x anti-bovine IgG conjugate PG.02: 1 0.3 ml bottle
Conjugate dilution buffer 3b: 1 25 ml bottle
Substrate solution 4b: 1 25 ml bottle
Stop solution 5b: 1 25 ml bottle
Negative serum -: 1 ml
Positive serum +: 1 ml
Description of the parts of the kit
*2x dilution buffer:*

- 2.5% casein pH 7
- 1x phosphate buffered saline (PBS)
- Yellow food dye
- 1:1000 Proclin300
- 2% saccharose

*20x wash buffer 2:*

- NaCl
- 0.1 M EDTA
- 10% Tween20
- 2 M Imidazole

*Conjugate*
*Conjugate dilution buffer 3b:*

- 2.5% casein pH 7
- 1x phosphate buffered saline (PBS)
- 1:1000 Proclin300
- Red food dye

*Substrate: Ready to use TMB (3, 3', 5, 5'-tetramethylbenzidine) solution*
*Stop solution: 0.2 M sulphuric acid*
*Control sera*
All control sera derive from experimentally infected animals.

Liquid reagent preparation

- Each liquid reagent is prepared so as to provide the use for a sufficient number of kits and identified with a lot number that shows the preparation date, the reagent and the kit ID and the operator ID.
- Each reagent is maintained at 4°C.
- Each reagent is aliquoted in bottles when assembling the kit.

The in vitro method for immunologically identifying a bovine herpes virus 1 (BoHV1) infection according to the present invention comprises the following steps.

In a first step, an aliquot of a biological sample from a bovine or a plurality of bovines is contacted with the support of the kit in conditions allowing the formation of an immunological complex formed by the gE and immunoglobulins possibly present in the biological sample. The first step has a duration from 45 minutes to 150 minutes, preferably from 50 minutes to 130 minutes, even more preferably from 60 minutes to 120 minutes.

If the biological sample is negative for the infection and contains no immunoglobulins directed to bovine herpes virus 1, no complex will be formed between the immunoglobulin in solution and the antigen immobilised on the support (the latter being the well of a microtitration plate in the above example). If, instead, the biological sample is positive for the infection and contains the above said immunoglobulins, an immunoglobulin-antigen complex will be formed and will be immobilised on the support.

The biological sample is preferably selected from the group consisting of blood serum, individual milk, pooled milk and immunoglobulin concentrate obtained from individual or pooled milk.

More preferably, the biological sample is individual milk or pooled milk.

Even more preferably, the biological sample is pooled milk deriving from a plurality of lactating bovines.

In a second step, the presence of the immunological complex is identified.

This can occur in different ways known to the skilled person, in particular by the use of a secondary anti-bovine IgG immunoglobulin conjugated with a compound that directly or indirectly develops a colorimetric reaction.

When the biological sample is pooled milk, i.e. a mixture of milk from a plurality of lactating bovines, the method preferably also comprises a step of concentrating the antibodies in the biological sample, preceding the step of contacting an aliquot of a biological sample with the support of the kit.

The concentration and purification of the immunoglobulins may be performed by different techniques known to the skilled in the art, e.g. using affinity chromatography, exploiting the binding of immunoglobulins with certain ligands (protein G, protein A etc.) A specific non-limitative example of the method according to the present invention is disclosed hereinafter.

Plates sensitised with recombinant BoHV1 gE on odd rows and with negative antigen on even rows are used.

The biological samples are used undiluted (milk) or diluted 1:2 (concentrated IgGs from milk) or diluted 1:20 (blood serum) directly on the plate and incubated for 120 minutes (milk) or 60 minutes (concentrated IgGs from milk and blood serum) at a room temperature.

After washing the secondary anti-bovine IgG immunoglobulin marked with peroxidase is added and the plate is incubated for 45 minutes at a room temperature.

After washing again, the substrate is added.

The colorimetric reaction is read after 15 minutes with a spectrophotometer. The intensity of the colorimetric reaction is proportional to the concentration of specific antibodies.

With reference to Figure 1 and depending on whether the biological sample is individual milk, concentrated milk IgGs from pooled milk or blood serum, the protocol varies as follows.

*Protocol 1 (milk)*

1. Dispense a 100 μl of 2x dilution buffer (1d) in wells A1, A2, B1 and B2.
2. Dispense 200 μl of the milk samples in wells C1 and C2 proceeding according to the plate pattern.
3. Incubate for 60 minutes at a room temperature.
4. Add a 100 μl of negative control in wells A1 and A2, a 100 μl of positive control in wells B1 and B2 and continue incubation for another 60 minutes at a room temperature.

*Protocol 2 (concentrated IgGs from pooled milk)*

1. Dispense a 100 μl of 2X dilution buffer (1d) in all the wells.
2. Add a 100 μl of negative control in wells A1 and A2, a 100 μl of positive control in wells B1 and B2.
3. Dispense a 100 μl of concentrated IgGs from milk in wells C1 and C2 proceeding according to the plate pattern.
4. Incubate the plate for 60 ± 5 minutes at a room temperature.

*Protocol 3 (blood serum)*

1. Dispense a 100 μl of 2x dilution buffer (1d) in wells A1, A2, B1 and B2.
2. Dispense a 190 μl of 1x dilution buffer in wells C1 and C2 proceeding according to the plate pattern (for sera: dilute the 2x diluent 1:2 with pure water).
3. Add a 100 μl of negative control in wells A1 and A2, a 100 μl of positive control in wells B1 and B2 and 10 μl of sample serum in wells C1 and C2 proceeding according to the plate pattern.
4. Incubate the plate for 60 ± 5 minutes at a room temperature.

The following steps of the method are common to the three protocols.

5. Dilute the washing buffer (2) 1:20 with pure water.
Example: for a plate, 10 ml of concentrated solution and a 190 ml of water.
6. Wash the plate 4 times with 300 μl/well. Add the washing buffer, wait 30 seconds, empty the plate by inversion; repeat again twice. At the end of the last wash, drying must be completed by tilting the plate and dabbing with paper.
7. Dilute the 100x concentrated conjugate (PG.02) in the conjugate dilution buffer. Example: for a plate, 120 μl of 100x conjugate in 11.88 ml of conjugate dilution buffer.
8. Add 100 μl of diluted conjugate in each well.
9. Incubate the plate for 45 ± 5 minutes at a room temperature. During incubation, maintain the substrate at a room temperature (solution 4).
10. Wash the plate 3 times with 300 μl/well.
11. Add 100 μl of substrate (4b) in each well.
12. Incubate the plate at a room temperature for 15 min.
13. Block the colorimetric reaction by adding 100 μl of stop solution (5b) in each well.
14. Read the plate at 450 nm.

Computation and Interpretation

Compute the net absorbance (nOD) of each sample and of the controls by subtracting the absorbance of the negative antigen (even rows) from the absorbance of the positive antigen (odd rows) and apply the following formula:

$$\frac{\text{nOD sample}}{\text{nOD pos C}} \times 100$$

Samples having a reactivity ≥ 40% are considered positive.
Samples having a reactivity ≥ 30% were considered negative.
Samples having a reactivity from 30% to 40% were considered uncertain.

Validation:

The net absorbance of the positive control must be > 0.4.
The ratio between net absorbances of the positive control and the negative control must be > 3.

Examples

The kit and the method according to the invention and of the above disclosed preferred embodiment were validated on serum, individual milk and pooled milk subjected to concentration of the IgGs.
In the following examples the kit according to the invention will also be referred to as "indirect ELISA kit" in order to distinguish it from the kit of EP0587805 which will also be referred to as "competitive ELISA kit".

Example 1: Milk

66 milk samples from negative individuals and 11 milk samples from positive individuals were tested. All samples subjected to the indirect ELISA test of protocol 1, were correctly classified.

Example 2: Concentrated IgGs from pooled milk

In this case a protocol for the concentration of IgGs was used, which includes the following steps.

1. The pooled milk sample is subjected to the precipitation of caseins by means of rennet coagulation and the milk serum is transferred to a new tube.
2. The addition of the affinity matrix binds the IgGs (e.g.: Protein G sepharose).
3. The matrix/IgG complex is pelleted by centrifugation, resuspended in washing buffer and transferred on a microcentrifuge column.
4. After an additional wash, the IgGs are eluted in a small volume (200 $\mu$l) and neutralised with 20 $\mu$l of Tris 1.5 M pH 7.5.
5. The resulting sample can be directly used in the serological test.
6. The kit contains a reagent for protein quantification by means of a colorimetric method for verifying each single extraction.

The routine use of this extraction method allows to establish that a sample concentration of 2 mg/ml and a purity degree of 95% as assessed by SDS-PAGE are appropriate to obtain the performance of sensitivity stated by the inventors in the indirect ELISA test. Methods for the extraction and/or concentration and/or purification of the immunoglobulins other than those used in the present example must meet the same concentration and purity requirements.
For the validation of this matrix, milk samples from animals positive to the competitive gE ELISA test and negative milk samples from officially disease-free farms were used. The positive milk was diluted in the negative milk by using the following dilutions: 1:5, 1:10, 1:20, 1:30, 1:40. IgGs were concentrated/purified by the disclosed method from 10 ml of milk obtained in each dilution. The concentrate was used with the indirect ELISA test according to the instructions of protocol 2.
The graph of Figure 2 shows that by mixing a positive milk volume with 39 negative milk volumes (1:40 dilution) and subjecting 10 ml of this mixture to the above disclosed immunoglobulin purification/concentration protocol, a positive result is obtained (dotted line). These data correspond to a prevalence of infection of 2.5% which is appropriate to detect even a small viral circulation in farms where vaccination with a marker vaccine has been carried out.
After this laboratory test, pooled milk samples from 76 farms were tested, the farms having different health status for BoHV1 infection. Precisely:

- 47 farms were BoHV1 negative and included 4765 lactating animals. 18 were officially disease-free (negative without the use of marker vaccines) and 29 were disease-free (marker vaccinated and gE negative).
- 29 farms were infected (gE positive animals).

All disease-free and officially disease-free farms except one (which will be discussed in detail hereinafter), were negative to the serological test (data not shown), whereas positive farms in which prevalence was higher than 2.5% except two were positive. The first included lactating Holstein Friesian cattle negative to the competitive gE test and seropositive Piedmontese cattle which were positive but were kept in a separate shed. The second included Holstein Friesian cows, some of which had been vaccinated in the past with a traditional inactivated vaccine (no marker). It is therefore clear that, except for these cases, which do not derive from a viral circulation, the test potentially allows to detect a prevalence of 2.5% even in field conditions.
A further confirmation of the ability to detect a recent viral circulation is provided by the single farm which was officially disease-free and resulted positive to the indirect ELISA test. The farm at issue recently obtained the "officially disease-free" certification and included 64 lactating cows. The farm was double-checked in the subsequent month by considering all individual milks, pooled milk and pooled milk concentrated and purified as disclosed above.

As can be noted from the graph in Figure 4, individual milk sample 1 resulted positive among the 64 individual milk samples. The head was subsequently confirmed to be positive by the competitive gE test on blood serum.

Further inquiries clarified that the animal had been vaccinated 10 years earlier with a traditional attenuated vaccine (no marker). In the course of time, the animal had become negative, allowing the farm to obtain the "officially disease-free" certification even without its elimination.

The reactivation of the vaccine strain without excretive phase resulted in the reappearance of antibodies in the same subject by booster effect.

The pooled milk tested with the ELISA resulted negative, whereas the same sample concentrated and purified with the above disclosed method was negative, showing that the method allowed to detect a positive subject among 64 cows.

Example 3 (serum)

34 positive samples and 254 samples negative to the competitive gE ELISA test were tested. The samples were subjected to the indirect ELISA test with protocol 3. The results obtained with the indirect ELISA test showed a concordance with the results of the competitive ELISA equivalent to 0.90 (Cohen's Kappa, 95%CI 0.82 - 0.98). The achieved specificity was 98.82% (95%CI 96.59% - 99.75%). The sensitivity values are not reported.

Example 4

The reactivity of a panel of monoclonal antibodies against native and recombinant antigens (including those used in two commercial competitive kits) was assessed. The results are summarised in following Table 1

Table 1

| | 2A5 Anti BoHV1 gE | 5H5 Anti BoHV1 gI | Mab-gE competitive ELISA (commercial) mAb67 | Mab-gE competitive ELISA (commercial) Hipra CIVTEST BOVIS IBRgE | 5F5 Anti BoHV1 gE | 4D7 Anti BoHV1 gE |
|---|---|---|---|---|---|---|
| BoHV1 field strain | + | + | + | + | + | + |
| BoHV1 ΔgE strain | - | + | - | - | - | - |
| pSEC/Bo-gI | - | + | - | - | - | - |
| pSEC/Bo-gE | + | - | - | - | + | + |
| pSEC/Bo-gE+gI | + | + | ± | ± | + | + |

By ± there is intended that a weak reactivity was obtained.

Antibodies 2A5, 5F5 and 4D7 are disclosed in Egyed et al., Acta Vet. Hung. 40:225-230.

The mAb 67 antibody is disclosed in Van Oirschot et al., J Virol Methods; 67:23-34.

From the above results it is clear that:

- the commercial kit antibodies recognise epitopes on the gI/gE complex and do not recognise the gE on its own;
- the use of the recombinant gE/gI heterodimer is inefficient in the competitive test (see last line). The result is that the use of a recombinant gE/gI heterodimer does not overcome the drawback of false positives due to steric hindrance;
- the recombinant gE has at least 3 distinct epitopes which bind at least 3 different anti-gE immunoglobulins.

Example 5

Figure 5 shows the results of experiments in which the reactivity of sera, which were found to be false positives when tested by a commercial competitive test, was evaluated. The reactivity to the recombinant gE in the indirect ELISA is shown on the x axis, whereas the inhibition percentage in the competitive ELISA is shown on the y axis. The horizontal and vertical lines represent the cutoff of the indirect and competitive test.

The assay according to the present invention therefore overcomes the specificity problems related to the steric hindrance of commercial assays of the prior art.

Example 6

The results of seroconversion experiments in experimentally infected animals are shown in Figure 6. The upper line in the graph represents the reactivity of the sera in the indirect ELISA, whereas the lower line represents the reactivity of the sera in the competitive ELISA. The horizontal band represents the area in which the result in uncertain.

The indirect test therefore has advantages in terms of an earlier diagnosis, as it can detect a recent infection significantly earlier, as shown in the experimental infections of this example.

From the above reported data, the advantages the kit and method according to the invention allow to obtain are clear.

In particular, the kit is more sensitive than the commercially available kits. One of the reasons of the higher sensitivity is that, while the kits according to the prior art comprised a monoclonal antibody directed to a single immunodominant epitope of the gE, the recombinant glycoprotein E of SEQ ID NO:1 has different epitopes which can bind the different immunoglobulins.

In virtue of the fact that the recombinant gE immobilised on the support of the kit has at least two different epitopes binding at least two different anti-gE immunoglobulins, preferably three different epitopes binding at least three different anti-gE immunoglobulins, the kit represents an independent test with respect to the traditional competitive test in which a gE/gI epitope is recognised. The fact that the epitopes all belong to glycoprotein E and not to the gE/gI complex results in the test not being negatively affected by the steric hindrance of gI and therefore resulting more specific. These data are further confirmed by the analysis of the recombinant antigen with the panel of monoclonal antibodies directed to the BoHV1 native gE of Example 4, in which at least three distinct epitopes (a linear epitope and two conformational epitopes) were shown to be present in the protein immobilised in the solid phase.

The kit according to the invention can therefore detect several antibodies in the tested biological sample.

Further, the kit according to the invention comprises a limited number of parts and is therefore cost-effective to produce.

The method according to the invention is also simple and does not require a highly qualified operator.

Furthermore, in virtue of the fact that the step of contacting an aliquot of a biological sample with the support of the kit has a duration from 45 minutes to 150 minutes, the method is particularly rapid and allows a diagnosis in a short time.

Furthermore, in virtue of the high sensitivity of the kit, the kit and the method according to the invention can be applied both to individual milk and to pooled milk. In particular, the application of the kit and the method to pooled milk with or without a prior step of concentrating IgGs allow a low-cost screening for milk-producing farms. Thereby, the need for veterinary-performed blood sampling as well as the costs for analyses are decreases. The individual serological analysis is performed only in case positivity is confirmed.

The application of the indirect ELISA test on blood serum is useful even if the concordance with the competitive test is not complete. The indirect ELISA test is independent with respect to the competitive test and can detect antibodies directed to different epitopes of the gE and epitopes other than that competing with the monoclonal antibody of the competitive test of EP0587805. The possible reasons for false positive results are different in the two tests (steric hindrance in the competitive test, nonspecific binding of the serum to heterologous proteins in the indirect test). As a consequence, the sequential application of the two tests (competitive test as screening and indirect test where diagnosis needs to be confirmed) improves the performances of the diagnostic process for the control of IBR, thus enormously increasing the positive predictive value of the diagnostic protocol.

As reported in Tyborowska et al., J Virol Methods, 145(2): 333-351, glycoprotein I is required to detect reactivity in the prior art competitive assay and the monoclonal antibody used in the assay does not recognize recombinant gE on its own. This is the reason of the independent nature of the assay according to the invention. On the other hand, gI, although less immunogenic than other viral glycoproteins, for its proximity to gE, may be responsible for potential false positive reactions in the competitive ELISA when hyper-vaccinated animals are tested shortly after vaccination, due to steric hindrance.

In sequential testing, when positives from the assay of the prior art are further tested with the assay of the present invention, the net effect is to increase specificity and positive predictive value because each case has to test positive to multiple tests (so false positives are rare). For this reason, an independent test should be used to confirm the positivity obtained using a blocking ELISA for screening, especially during the last eradication phases. Indeed, in scenarios where disease prevalence is low (lower than 5%), the assay according to the present invention can reach high positive and negative predictive values (94.5% and 99.9% respectively), supporting the decision on the positivity of the tested samples.

## Claims

1. A kit comprising a support on which a recombinant bovine herpes virus 1 (BoHV1) glycoprotein E (gE) is immobilised, the recombinant BoHV1 gE comprising at least two different epitopes which respectively bind at least two different anti-glycoprotein E immunoglobulins.

2. The kit according to claim 1, comprising at least three different epitopes which respectively bind at least three different anti-glycoprotein E immunoglobulins.

3. The kit according to claim 1 or 2, wherein the recombinant BoHV1 gE comprises SEQ ID NO:1.

4. The kit according to claim 3, wherein the recombinant BoHV1 gE has SEQ ID NO:1.

5. The kit according to any of the preceding claims, wherein the recombinant BoHV1 gE is produced in mammal cells.

6. The kit according to any of the preceding claims, wherein the support is an ELISA microtitration plate.

7. The kit according to claim 6, wherein the ELISA microtitration plate is a 96 well plate and the amount of recombinant gE immobilised per well is in the range from 0.025 μg to 0.2 μg.

8. The kit according to any of the preceding claims, wherein at least one other recombinant glycoprotein E is immobilised on the support, the at least one other recombinant glycoprotein E belonging to a herpes virus other than bovine herpes virus 1 (BoHV1).

9. An in vitro method for immunologically identifying a bovine herpes virus 1 (BoHV1) infection comprising the steps of:

- contacting an aliquot of a biological sample from a bovine or a plurality of bovines with the support of the kit according to any of claims from 1 to 8 in conditions allowing the formation of an immunological complex formed by the gE and immunoglobulins possibly present in the biological sample;
- identifying the presence of the immunological complex.

10. The method according to claim 9, wherein the step of contacting an aliquot of a biological sample with the support of the kit has a duration from 45 minutes to 150 minutes.

11. The method according to claim 9 or 10, wherein the biological sample is selected from the group consisting of blood serum, individual milk, pooled milk and immunoglobulin concentrate obtained from individual or pooled milk.

12. The method according to claim 11, wherein the biological sample is individual milk or pooled milk.

13. The method according to claim 12, wherein the biological sample is pooled milk from a plurality of lactating bovines.

14. The method according to claim 12 or 13, wherein the method also comprises a step of concentrating immunoglobulins in the biological sample, preceding the step of contacting an aliquot of a biological sample with the support of the kit.

| | 1 | 2 | 3 | 4 | 5 | 6 | 7 | 8 | 9 | 10 | 11 | 12 |
|---|---|---|---|---|---|---|---|---|---|---|---|---|
| A | CNeg | CNeg | C7 | C7 | C15 | C15 | C23 | C23 | C31 | C31 | C39 | C39 |
| B | CPos | CPos | C8 | C8 | C16 | C16 | C24 | C24 | C32 | C32 | C40 | C40 |
| C | C1 | C1 | C9 | C9 | C17 | C17 | C25 | C25 | C33 | C33 | C41 | C41 |
| D | C2 | C2 | C10 | C10 | C18 | C18 | C26 | C26 | C34 | C34 | C42 | C42 |
| E | C3 | C3 | C11 | C11 | C19 | C19 | C27 | C27 | C35 | C35 | C43 | C43 |
| F | C4 | C4 | C12 | C12 | C20 | C20 | C28 | C28 | C36 | C36 | C44 | C44 |
| G | C5 | C5 | C13 | C13 | C21 | C21 | C29 | C29 | C37 | C37 | C45 | C45 |
| H | C6 | C6 | C14 | C14 | C22 | C22 | C30 | C30 | C38 | C38 | C46 | C46 |

FIG. 1

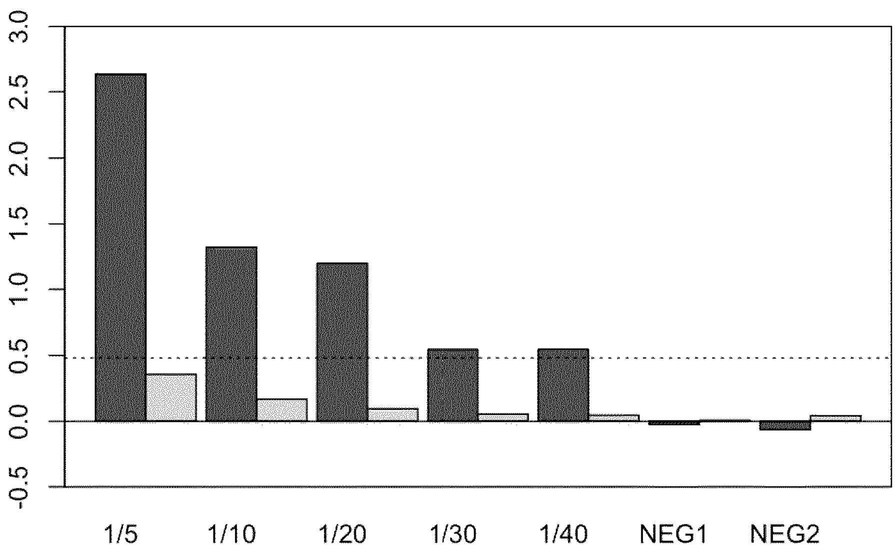


FIG. 2

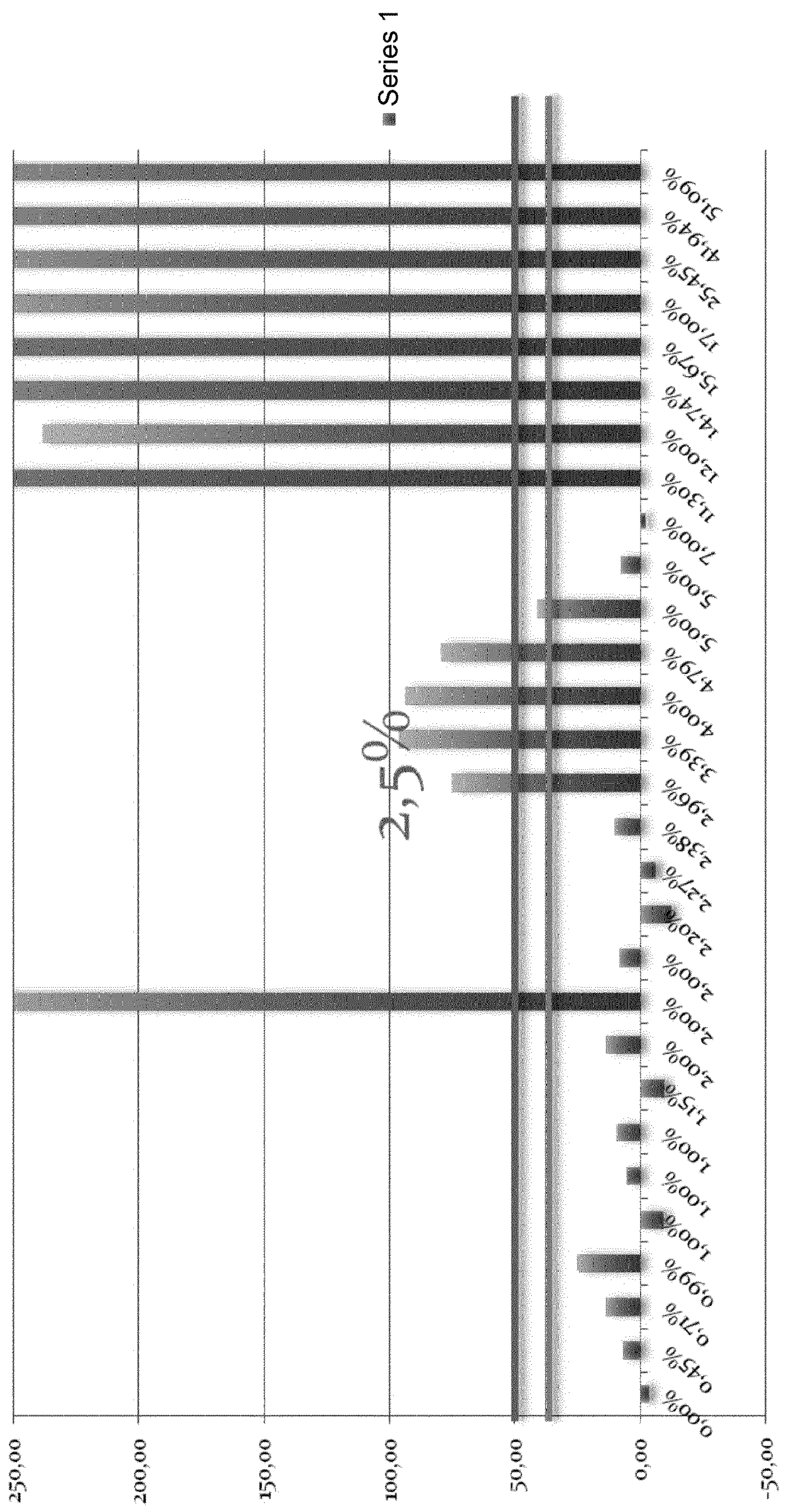
Series 1
2,5%
250,00
200,00
150,00
100,00
50,00
0,00
-50,00
0,00%
0,45%
0,71%
0,99%
1,00%
1,00%
1,00%
1,15%
2,00%
2,00%
2,00%
2,20%
2,27%
2,38%
2,96%
3,39%
4,00%
4,79%
5,00%
5,00%
7,00%
11,30%
12,00%
14,74%
15,67%
17,00%
25,45%
41,94%
51,09%

FIG. 3

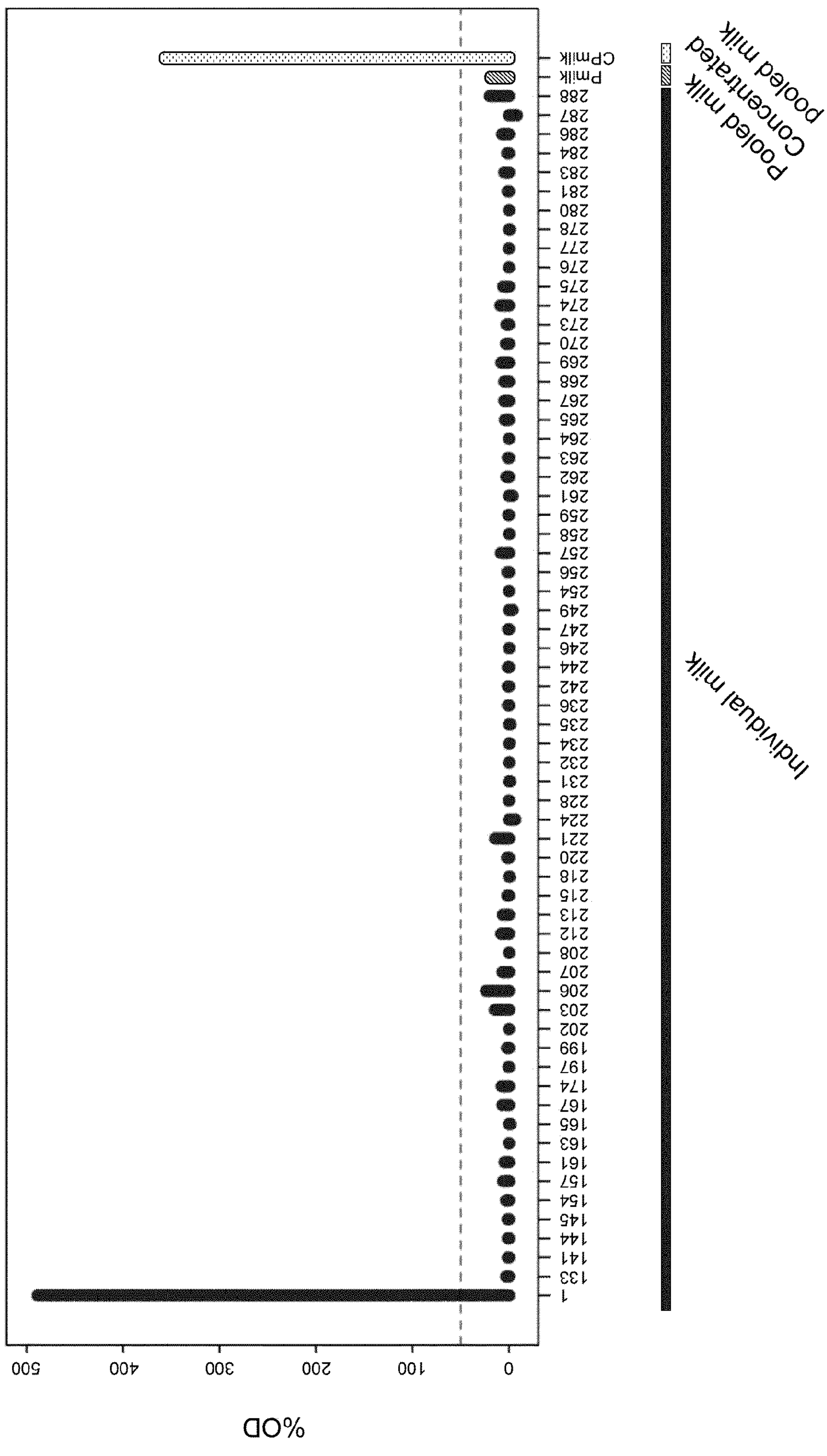
%OD
0
100
200
300
400
500
1
133
141
144
145
154
157
161
163
165
167
174
197
199
202
203
206
207
208
212
213
215
218
220
221
224
228
231
232
234
235
236
242
244
246
247
249
254
256
257
258
259
261
262
263
264
265
267
268
269
270
273
274
275
276
277
278
280
281
283
284
286
287
288
Pmilk
CPmilk
Individual milk
Pooled milk
Concentrated pooled milk

FIG. 4

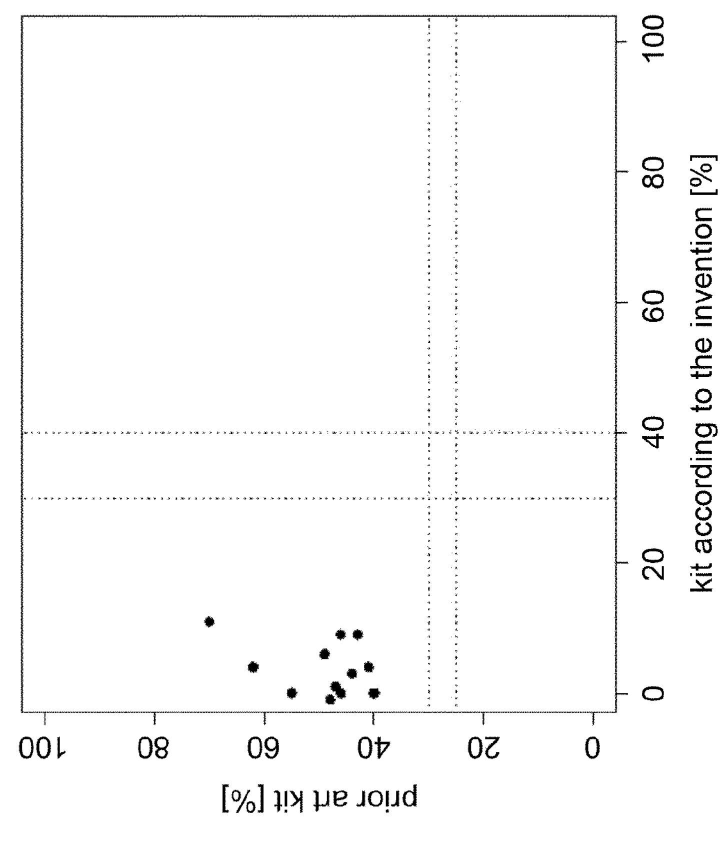
prior art kit [%]
kit according to the invention [%]
0
20
40
60
80
100

FIG. 5

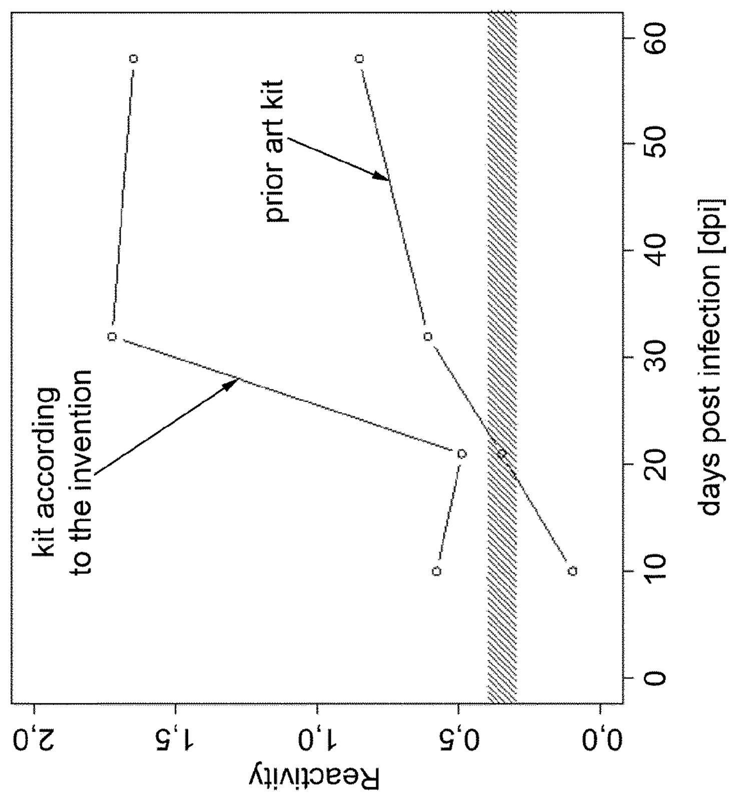
Reactivity
days post infection [dpi]
kit according
to the invention
prior art kit
0,0
0,5
1,0
1,5
2,0
0
10
20
30
40
50
60

FIG. 6

Europäisches Patentamt
European Patent Office
Office européen des brevets

# EUROPEAN SEARCH REPORT

Application Number
EP 15 16 7043

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAN OIRSCHOT J ET AL: "An enzyme-linked immunosorbent assay to detect antibodies against glycoprotein gE of bovine herpesvirus 1 allows differentiation between infected and vaccinated cattle", JOURNAL OF VIROLOGICAL METHODS, ELSEVIER BV, NL, vol. 67, no. 1, 1 August 1997 (1997-08-01), pages 23-34, XP026020894, ISSN: 0166-0934, DOI: 10.1016/S0166-0934(97)00073-6 [retrieved on 1997-08-01] * the whole document * * abstract * * pages 24-33; figures 1-7 * ----- | 1-14 | INV. G01N33/569 C07K14/005 C12N7/00 C12N15/86 |
| X | C LETELLIER ET AL: "Characterization of monoclonal antibodies directed against the bovine herpesvirus-1 glycoprotein E and use for the differentiation between vaccinated and infected animals", VETERINARY MICROBIOLOGY, vol. 83, no. 4, 1 December 2001 (2001-12-01), pages 301-315, XP055160375, ISSN: 0378-1135, DOI: 10.1016/S0378-1135(01)00430-8 * the whole document * * abstract * * pages 301-302 * * point 2.5-2.9; page 304 - page 306; table 2 * * point 3.1-3.6; pages 306-311; figures 2,3; tables 3-5 * ----- | 1-14 | TECHNICAL FIELDS SEARCHED (IPC): G01N C07K C12N |
| | -/-- | | |

The present search report has been drawn up for all claims

1

| Place of search | Date of completion of the search | Examiner |
|---|---|---|
| The Hague | 25 August 2015 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 16 7043

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ALAA A. EL-KHOLY ET AL: "Baculovirus expression and diagnostic utility of the glycoprotein E of bovine herpesvirus-1.1 Egyptian strain "Abu-Hammad"", JOURNAL OF VIROLOGICAL METHODS, vol. 191, no. 1, 1 July 2013 (2013-07-01), pages 33-40, XP055160128, ISSN: 0166-0934, DOI: 10.1016/j.jviromet.2013.03.024 * the whole document * * abstract * ----- | 1-14 | |
| Y | LUIGI BERTOLOTTI ET AL: "Characterization of caprine herpesvirus 1 (CpHV1) glycoprotein E and glycoprotein I ectodomains expressed in mammalian cells", VETERINARY MICROBIOLOGY, vol. 164, no. 3-4, 1 June 2013 (2013-06-01), pages 222-228, XP055160082, ISSN: 0378-1135, DOI: 10.1016/j.vetmic.2013.02.008 * the whole document * * abstract * ----- | 1-14 | TECHNICAL FIELDS SEARCHED (IPC) |
| Y | G.J WELLENBERG ET AL: "Antibodies against bovine herpesvirus (BHV) 5 may be differentiated from antibodies against BHV1 in a BHV1 glycoprotein E blocking ELISA", VETERINARY MICROBIOLOGY, vol. 78, no. 1, 1 January 2001 (2001-01-01), pages 79-84, XP055160137, ISSN: 0378-1135, DOI: 10.1016/S0378-1135(00)00283-2 * the whole document * * abstract * ----- -/-- | 1-14 | |

The present search report has been drawn up for all claims

1

| Place of search | Date of completion of the search | Examiner |
|---|---|---|
| The Hague | 25 August 2015 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 16 7043

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | LEHMANN D ET AL: "Characterization of BoHV-1 gE envelope glycoprotein mimotopes obtained by phage display", VETERINARY MICROBIOLOGY, ELSEVIER BV, NL, vol. 104, no. 1-2, 30 November 2004 (2004-11-30), pages 1-17, XP004629356, ISSN: 0378-1135, DOI: 10.1016/J.VETMIC.2004.08.012 * the whole document * * abstract * ----- | 1-14 | |
| Y | F. A. M. RIJSEWIJK ET AL: "Epitopes on glycoprotein E and on the glycoprotein E/glycoprotein I complex of bovine herpesvirus 1 are expressed by all of 222 isolates and 11 vaccine strains", ARCHIVES OF VIROLOGY, vol. 145, no. 5, 1 May 2000 (2000-05-01), pages 921-936, XP055160140, ISSN: 0304-8608, DOI: 10.1007/s007050050684 * the whole document * ----- | 1-14 | TECHNICAL FIELDS SEARCHED (IPC) |
| Y | J. TYBOROWSKA ET AL: "The extracellular part of glycoprotein E of bovine herpesvirus 1 is sufficient for complex formation with glycoprotein I but not for cell-to-cell spread", ARCHIVES OF VIROLOGY, vol. 145, no. 2, 15 February 2000 (2000-02-15), pages 333-351, XP055160144, ISSN: 0304-8608, DOI: 10.1007/s007050050026 * the whole document * ----- | 1-14 | |

1

The present search report has been drawn up for all claims

| Place of search | Date of completion of the search | Examiner |
|---|---|---|
| The Hague | 25 August 2015 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0587805 A

### Non-patent literature cited in the description


- **EGYED et al.** *Acta Vet. Hung.,* vol. 40, 225-230
- **VAN OIRSCHOT et al.** *J Virol Methods,* vol. 67, 23-34
- **TYBOROWSKA et al.** *J Virol Methods,* vol. 145 (2), 333-351

```
SEQUENCE LISTING

<110> In3diagnostic s.r.l.

<120> KIT AND IN VITRO METHOD FOR IDENTIFYING BOVINE HERPES VIRUS 1

<160> 2

<170> BiSSAP 1.0

<210> 1
<211> 386
<212> PRT
<213> Bovine herpesvirus 1

<220>
<221> SOURCE
<222> 1..386
<223> /mol_type="protein"
      /organism="Bovine herpesvirus 1"

<400> 1
Asp Pro Lys Pro Ala Thr Glu Thr Pro Gly Ser Ala Ser Val Asp Thr
1               5                   10                  15
Val Phe Thr Ala Arg Ala Gly Ala Pro Val Phe Leu Pro Gly Pro Ala
            20                  25                  30
Ala Arg Pro Asp Val Arg Ala Val Arg Gly Trp Ser Val Leu Ala Gly
        35                  40                  45
Ala Cys Ser Pro Pro Val Pro Glu Pro Val Cys Leu Asp Asp Arg Glu
    50                  55                  60
Cys Phe Thr Asp Val Ala Leu Asp Ala Ala Cys Leu Arg Thr Ala Arg
65                  70                  75                  80
Val Ala Pro Leu Ala Ile Ala Glu Leu Ala Glu Arg Pro Asp Ser Thr
                85                  90                  95
Gly Asp Lys Glu Phe Val Leu Ala Asp Pro His Val Ser Ala Gln Leu
            100                 105                 110
Gly Arg Asn Ala Thr Gly Val Leu Ile Ala Ala Ala Ala Glu Glu Asp
        115                 120                 125
Gly Gly Val Tyr Phe Leu Tyr Asp Arg Leu Ile Gly Asp Ala Gly Asp
    130                 135                 140
Glu Glu Thr Gln Leu Ala Leu Thr Leu Gln Val Ala Thr Ala Gly Ala
145                 150                 155                 160
Gln Gly Ala Ala Arg Asp Glu Glu Arg Glu Pro Ala Thr Gly Pro Thr
                165                 170                 175
Pro Gly Pro Pro Pro His Arg Thr Thr Thr Arg Ala Pro Pro Arg Arg
            180                 185                 190
His Gly Ala Arg Phe Arg Val Leu Pro Tyr His Ser His Val Tyr Thr
        195                 200                 205
Pro Gly Asp Ser Phe Leu Leu Ser Val Arg Leu Gln Ser Glu Phe Phe
    210                 215                 220
Asp Glu Ala Pro Phe Ser Ala Ser Ile Asp Trp Tyr Phe Leu Arg Thr
225                 230                 235                 240
Ala Gly Asp Cys Ala Leu Ile Arg Ile Tyr Glu Thr Cys Ile Phe His
                245                 250                 255
Pro Glu Ala Pro Ala Cys Leu His Pro Ala Asp Ala Gln Cys Ser Phe
            260                 265                 270
Ala Ser Pro Tyr Arg Ser Glu Thr Val Tyr Ser Arg Leu Tyr Glu Gln
        275                 280                 285
Cys Arg Pro Asp Pro Ala Gly Arg Trp Pro His Glu Cys Glu Gly Ala
    290                 295                 300
Ala Tyr Ala Ala Pro Val Ala His Leu Arg Pro Ala Asn Asn Ser Val
305                 310                 315                 320
Asp Leu Val Phe Asp Asp Ala Pro Ala Ala Ala Ser Gly Leu Tyr Val
                325                 330                 335
Phe Val Leu Gln Tyr Asn Gly His Val Glu Ala Trp Asp Tyr Ser Leu
            340                 345                 350
Val Val Thr Ser Asp Arg Leu Val Arg Ala Val Thr Asp His Thr Arg
        355                 360                 365
```

```
Pro Glu Ala Ala Ala Ala Asp Ala Pro Glu Pro Gly Pro Pro Leu Thr
    370                 375                 380
Ser Glu
385

<210> 2
<211> 1159
<212> DNA
<213> Bovine herpesvirus 1

<220>
<221> source
<222> 1..1159
<223> /mol_type="DNA"
      /organism="Bovine herpesvirus 1"

<400> 2
gatcccaaac cggccaccga gacacccggt tctgccagtg ttgacaccgt ctttactgcg      60

agagcaggcg cgccagtgtt tcttcccgga cccgcagcca gacccgatgt gagggccgtc     120

cggggttgga gtgtgcttgc tggagcatgc tctccaccgg tacctgaacc cgtctgtctg     180

gatgatcgcg aatgctttac cgatgtggcc ttggatgcag cctgtctgcg aaccgctcgg     240

gttgctcctc tcgccattgc cgagctggca gaaagaccag actcaacggg cgacaaggag     300

tttgtactcg ccgatcctca tgtgtccgca cagctgggga gaaatgccac aggggttttg     360

attgccgctg cagccgaaga ggacggcggc gtttacttcc tgtatgatag gctgatcggc     420

gacgcaggcg acgaagagac acagttggct ctgaccctgc aagtcgccac ggcaggcgct     480

caaggggctg ccagggatga ggagcgtgaa cccgccactg ggcctacccc gggacctcca     540

ccacatcgga ctaccactcg cgctccaccg cgcagacatg gcgcgcggtt ccgtgtgctg     600

ccttaccaca gccacgtgta tacaccgggt gactccttcc tgctctccgt gaggcttcag     660

tccgagttct tcgatgaagc tcctttcagt gccagcattg actggtactt cctccgaacg     720

gccggtgact gtgccctgat acgcatctac gagacttgca tctttcaccc cgaagcgccc     780

gcctgtctgc acccagccga cgcacagtgc agctttgcta gcccctacag gtcagagaca     840

gtctacagcc gcctgtatga gcagtgtcga cccgatcctg cgggaagatg gccacacgag     900

tgcgaaggag cggcttatgc agccccagtg gcccatctgc ggcctgccaa caacagcgta     960

gaccttgtct ttgacgatgc acccgcagct gcctcagggc tgtatgtgtt cgtgctgcag    1020

tacaatggac acgtggaggc ttgggactat tctctcgtcg tgacatctga taggctcgtt    1080

cgggccgtga ctgaccatac acgcccagaa gctgcggctg ctgacgcacc tgaacctggg    1140

ccacccttga cctccgaga                                                 1159
```